# EUROPEAN PATENT APPLICATION

(11) **EP 4 744 645 A1**
(43) Date of publication of application: **20.05.2026**
(21) Application number: 25383258.8
(22) Date of filing: 14.11.2025
(51) Int. Cl.: A61K 8/14, A61K 8/64, A61K 8/67, A61Q 19/00, A61Q 19/08

(54) **COSMETIC PREPARATION FOR REJUVENATING THE SKIN**

(30) Priority: 15.11.2024 EP 24383242
(71) Applicant: Prima-Derm, S.L., 08850 Gavà (ES)
(72) Inventor: SEGOVIA LASERNA, María del Rosario, 08850 GAVÀ (ES); BERMÚDEZ HERROJO, Ignacio, 08850 GAVÀ (ES); JULIÀ MÖLLER, Ricard, 08017 BARCELONA (ES); REIG, Amanda, 08850 GAVÀ (ES)
(74) Representative: Sugrañes, S.L.P.

(57) **Abstract**

The present disclosure relates to a cosmetic composition for rejuvenating the skin comprising from 0,01% to 0,5% by weight with respect to the total weight of the composition of a precursor of retinoic acid selected from the group consisting of retinaldehyde, retinol, retinyl retinoate and retinyl esters, preferably retinaldehyde, from 0,0001% to 0,01% by weight with respect to the total weight of the composition of an anti-aging or antioxidant peptide, and from 0,0000005% to 0,000005% by weight with respect to the total weight of the composition of a growth factor, wherein the precursor of retinoic acid is encapsulated by an encapsulating agent comprising phospholipids. The present disclosure also relates to a method for rejuvenating the skin, comprising the topical application of the cosmetic composition disclosed herein and to a use of the cosmetic composition disclosed herein for rejuvenating the skin, by means of its topical application.

## Description

### TECHNICAL FIELD

The present disclosure relates to a cosmetic composition for rejuvenating the skin. The present disclosure also relates to a method for rejuvenating the skin and to uses of the compositions herein disclosed.

### BACKGROUND ART

The skin forms the outer layer of the human being and is one of the most important organs. It has a surface area of about 2 m² and weighs 4-5 kg (6% of the total body weight). It consists of three morphologically differentiated layers (epidermis, dermis, hypodermis) that perform different functions while interrelating with each other. Many of its physiological functions are determined by its stratified structure. The epidermis consists mainly of keratinocytes that undergo a process of differentiation into corneocytes during their migration from the basal layers to the outermost layer, the stratum corneum. These corneocytes together with the surrounding lipids provide the physical and chemical barrier function of the skin. The epidermis also contains melanocytes that synthesize melanin and transfer it to keratinocytes that migrate to the upper layers.

The dermis lies below the epidermis and supports the epidermis by interacting with it through the dermal-epidermal junction. The main cell type in the dermis is fibroblasts, which are responsible for the synthesis of extracellular matrix components such as collagen, elastin and glycosaminoglycans.

The skin plays an important role as a barrier that separates us from the external environment by regulating and preventing, among other things, water loss and the entry of microorganisms and xenobiotics. It relates us to the external environment and protects us. But it also has a great social impact since its appearance influences the social behavior of most individuals. Skin aging is a continuous process where the functional homeostasis of the skin is altered and different changes in its appearance appear.

Skin aging is a multifactorial process induced by both intrinsic and extrinsic causes. It results in phenotypic changes, as well as in the structure and functions of the components of the extracellular matrix such as collagen, elastin and glycosaminoglycans involved in providing strength, elasticity and hydration to the skin.

The morphological changes produced by aging are characterized by a thinner epidermis and dermis with a lower number of keratinocytes and fibroblasts. Thinning of the epidermis increases the aged appearance of the skin with the presence of fine wrinkles while compromising barrier function and increasing water loss. The thinning of the dermis due to a decrease in collagen and elastin fibers increases its fragility and results in a loss of firmness.

Retinoids are natural or synthetic molecules that have structural similarities to vitamin A (retinol), one of the essential nutrients for our body. Vitamin A plays a key role in several biological functions, including vision, growth and development, reproduction, proper functioning of the immune system, and contributes to the health of the skin and mucous membranes, helping to maintain their integrity and barrier function.

The human body does not produce vitamin A naturally, so it must be acquired through the diet in the form of preformed vitamin A, which is found in foods of animal origin such as liver, fish, eggs and dairy products, or in the form of provitamin A (carotenoids), present in orange, yellow and dark green fruits and vegetables, such as carrots, sweet potatoes, spinach and broccoli. A daily intake of 0.7 mg of vitamin A is recommended for women and 0.9 mg for adult men, while the tolerable upper intake level (UL) is 3 mg per day. Vitamin A deficiency is the leading cause of preventable blindness in children worldwide. In pregnant women, vitamin A deficiency causes night blindness and can contribute to maternal mortality. In addition, it can also damage the immune system, cause thyroid disorders and skin disorders. Conversely, excessive consumption can lead to hypervitaminosis, which can be highly toxic, especially before and during pregnancy due to possible teratogenic effects.

In relation to the skin, the effect of vitamin A attracted the attention of dermatologists because vitamin A deficiency in humans was found to produce diseases related to skin keratinization, such as acne, psoriasis, ichthyosis or lichen planus, among others. The following are some historical milestones in the use of retinoids in medicine for the management of skin diseases:
- In 1943, vitamin A began to be administered orally for the treatment of acne. The acid form of this vitamin, retinoic acid, also began to be synthesized and administered orally for the treatment of keratinization disorders, which resulted in a revolution in the management of these diseases.
- In 1969, topical retinoic acid was used in acne, with good results.
- In 1972, other synthetic derivatives with vitamin A analogous activity began to be investigated, seeking more specificity and fewer adverse effects: acitretin, tazarotene, adapalene and bexarotene.

Numerous vitamin A analogs are currently available in the art, although in most cases their use is limited to the medical setting. Only retinol, retinal and retinyl esters can be used for cosmetic purposes. When applied topically to the skin, retinoids can penetrate the epidermis and dermis and reach keratinocytes and fibroblasts, where they coexist and exert different functions in multiple cellular compartments. First, retinoid storage is mostly regulated in the cytoplasm of keratinocytes, where retinoids are transformed to retinyl esters by esterification reactions controlled by lecithin retinol acyltransferase (LRAT) and acyl-CoA retinol acyltransferase (ARAT). These stored retinyl esters can subsequently be converted back to retinol by esterases. Also in the cytoplasm, retinol undergoes a two-step conversion process to retinoic acid. In the first step, the enzyme alcohol dehydrogenase (ADH) catalyses the conversion of retinol to retinaldehyde, while, in the second step, retinaldehyde dehydrogenase (RALDH) converts retinaldehyde to retinoic acid.

Retinoids are among the most studied ingredients in skin care to combat aging and improve skin's appearance. Their effects have been demonstrated at both the epidermal and dermal levels, exerting their influence mainly on three key categories of skin cells: epidermal keratinocytes, dermal fibroblasts and endothelial cells. Its main beneficial effects are detailed below:
1. Topical application of retinoids has an impact on increasing the thickness of the epidermis. This is due to the increase in the expression of the c-Jun protein, which is one of the main components of the AP-1 transcription factor, which is of utmost importance in the proliferation of epidermal keratinocytes.
2. Stimulates the proliferation of endothelial cells and blood vessels in the dermis. This increased dermal vascularization may promote better circulation in the skin, creating a more conducive environment for epidermal and dermal homeostasis.
3. Retinols may trigger activation of the TGF-β/CTGF pathway, a key regulator of extracellular matrix balance, resulting in increased expression of type I collagen, the major structural protein of the skin. In addition to the increase in type I collagen, the expression of fibronectin and tropoelastin may also be enhanced.
4. Retinoids regulate melanogenesis by reducing the activity of tyrosinase, a key enzyme in melanin synthesis, decreasing melanin production and its transfer to keratinocytes.
5. Retinoids may exhibit anti-acne effects, due to their anti-inflammatory effects that include inhibition of bacteria-induced proinflammatory pathways, reduction in cytokine and nitric oxide release.

For instance, EP2649986 A2 discloses a cosmetic composition for rejuvenating the skin characterized in that it comprises a precursor of retinoic acid from the group made up of retinaldehyde, retinol, retinyl retinoate and retinyl esters, and melatonin.

US2011262373 A1 discloses a personalized pharmaceutical composition for skin rejuvenation comprising a first group of common active ingredients comprising retinoic acid, one or more anti-inflammatory agents, one or more depigmenting agents, one or more antioxidants, and one or more vitamins; and a second group of variable active ingredients, wherein at least one anti-inflammatory agent is indomethacin.

The European Commission's Scientific Committee on Consumer Safety (SCCS) published in October 2022 a final opinion concerning the limiting the use of retinol and retinol esters in cosmetic products marketed in the European Union as of June 2024, as concerns regarding the percentage of the population being exposed to doses of Vitamin A over the recommended overall daily intake allowances, either through diet, food supplements or cosmetic products, had grown.

The SCCS is of the opinion that vitamin A in cosmetics at concentrations of 0.05% retinol equivalent (RE) in body lotions and 0.3% RE in other leave-on and rinse-off products is safe. Therefore, use is limited to 0.3% retinol and retinol esters in non-rinse-off cosmetic products or product compositions. This limitation does not affect retinal or other molecules not specifically addressed in the SCCS Opinion.

In the context of the present invention, the terms retinaldehyde or retinal are used indistinctively. Retinaldehyde or retinal is a molecule that requires one step less of transformation to become retinoic acid, which could help to act faster on the skin. Conversely, retinaldehyde is generally less stable than retinol, which means it can degrade more quickly, thus providing stable skin composition still remain a challenge.

Although the potency of retinaldehyde is higher than retinol, the side effects may be greater in the case of subjects with sensitive skin. Thus, compositions which are suitable and non-irritant for all type of skins are highly desirable.

The efficacy of a cosmetic product or product composition depends largely on the characteristics of its formula and the concentration of the active ingredients it contains, as well as on the capacity of these active ingredients to reach the biological targets on which they must act. It is for this reason that delivery systems are today one of the greatest challenges in the cosmetic industry. Some delivery systems allow the improvement of the product composition efficacy by increasing the quantity of retinols use or by increasing the penetration of active ingredients into the skin.

As is well known, human skin acts as a barrier against the permeation of exogenous molecules. The outermost layer of the skin, the stratum corneum, is a complex structure formed by keratinized cells that are surrounded by a lipid matrix of varying composition. The lipids present in skin matrixes may be among others, ceramides, cholesterol, phospholipids, triglycerides, cholesterol, sphingolipids, wax esters or/and fatty acids.

For instance, US12059486 discloses a method to prepare a tocopherol phosphate mixture liposomal composition comprising, among other features, the mixture of a lipophilic ingredient that may be a retinoid, that is encapsulated in the liposome.

To date, cosmetic compositions for skin rejuvenation containing retinol in various forms have been disclosed. These compositions employ different delivery systems for the retinol. However, one of the challenges that remains is the development of a composition that is stable over time and effective, without causing undesirable side effects such as dryness or skin irritation.

Anonymous internet publication made in the MINTEL DATABASE GNPD, www.gnpd.com/, published on the 5^{th} February 2024, with Database accession no. 11426126 and entitled "3X Antioxidant Cream", described a cream composition which comprised a non-encapsulated retinol, EFG (a growth factor) and antioxidant peptides, such as Copper Tripeptide-1 or Acetyl Hexapeptide-8. "3X Antioxidant Cream" internet publication also disclosed that said cream further comprised hydrogenated lecithin, commonly used as a humectant to provide hydration or to improve the texture of cosmetic compositions. 3X Antioxidant Cream" disclosed that said cream showed a modest anti-wrinkle effect after two weeks, of use: 15.9% improvement rate in deep wrinkles, 12.7% improvement rate in fine wrinkles, 22.7% improvement rate in induced wrinkles, as well as moderate improvements in elasticity, of about 12.0% (around the cheeks/mouth area) or 6.98% improvement in elasticity up to the 15th layer of the skin.

Other anonymous internet publication made in the MINTEL DATABASE GNPD, www.gnpd.com/, published on the 28^{th} of December 2023, and entitled "Retinol Super Bounce Serum" (with Database accession no. 11289818) described a serum composition in which retinol was present in various forms, said disclosure claimed to provide a composition with renewing, firming and brightening properties to the skin. The various forms of retinol were present as a mixture of; pure retinal, encapsulated retinal, granactive retinoid and bio-seletinoid (retinoid), having a weight concentration of the retinol blend of 1%. Additionally, the composition also comprised other components such as growth factors, as sh-oligopeptide-1 or decapeptide-4, antiaging peptides, such as AcetylTetrapeptide-11 and hydrogenated phosphatidylcholine. Although the internet disclosure of "Retinol Super Bounce Serum" mentioned the presence of encapsulated retinol, it failed to disclose how the retinol was encapsulated, let alone if an encapsulating agent comprising phospholipids was used for said purpose. Hydrogenated phosphatidylcholine is not limited to its use in the formation of liposomes, but it is also used in cosmetic compositions as an emulsifier, a skin conditioning agent, and in enhancing skin hydration. The serum composition disclosed herein, showed a moderate anti-wrinkle effect, with - 10.6% wrinkles reduction lips, -8.5% wrinkles reductions in the smile line or a wrinkle around the eyes of -7.0% as well as modest Skin firmness 12.2%

Further anonymous internet publication made in the MINTEL DATABASE GNPD, www.gnpd.com/, published on 24^{th} of January 2023, with Database accession no. 10527986 and entitled "Youth Liposomal Mist", described anti-ageing rejuvenating liposomal mist compositions containing the combination of 3 three types of retinols: retinol, retinal y retinyl propionate. However, the quantity of the different types of retinols used in the composition is not disclosed. This liposomal mist composition further includes growth factors, such as Nicotiana Benthamiana Hexapeptide-40 sh-Polypeptide-76, but it does not contain any anti-aging or antioxidant peptides. The disclosure of "Youth Liposomal Mist" failed to specify whether the combination of these three retinols was encapsulated in phospholipids, nor does it provide information on the quantities of each component present in the composition.

There remains a need in the art for stable cosmetic compositions that minimize skin irritation and dryness, enhance skin penetration, provide good anti-wrinkle efficacy, luminosity effect, spots reduction on the skin and a good appearance of the pores of the skin and/or good skin elasticity and firmness upon application, while comprising low quantities of retinoids (w/w %), such as retinaldehyde.

### SUMMARY OF THE DISCLOSURE

One aspect of the present disclosure provides a cosmetic preparation for rejuvenating the skin comprising from 0,01% to 0,5% by weight with respect to the total weight of the composition of a precursor of retinoic acid, which can be selected from the group consisting of retinaldehyde, retinol, retinyl retinoate, retinyl esters and combinations thereof, from 0,0001% to 0,01% by weight with respect to the total weight of the composition of an anti-aging or antioxidant peptide, and from 0,0000005% to 0,000005% by weight with respect to the total weight of the composition of growth factor, wherein the precursor of retinoic acid is encapsulated by an encapsulating agent comprising phospholipids. Preferably, the precursor of retinoic acid is retinaldehyde.

A second aspect of the present disclosure provides a method for rejuvenating the skin, characterized in that it comprises the topical application of the cosmetic composition as disclosed herein.

A third aspect of the present disclosure relates to the use of the cosmetic composition as disclosed herein for rejuvenating the skin, by means of its topical application.

Other features of the invention are disclosed in the detailed description of the invention.

### BRIEF DESCRIPTION OF THE FIGURES

The foregoing and other advantages and features will be more fully understood from the following detailed description of an embodiment with reference to the accompanying drawings, to be taken in an illustrative and non-limitative manner, in which:

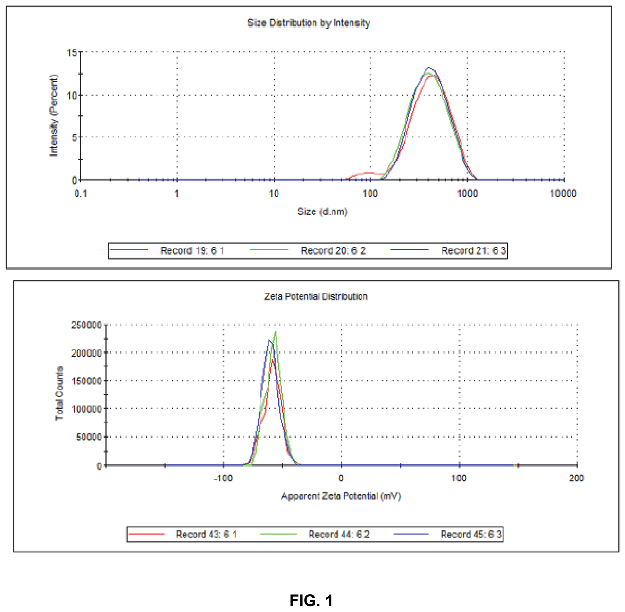

FIG. 1 accounts for two graphs illustrating the distribution of particle size of encapsulated retinal according to the invention, in three different samples (graph above) and the Z-potential obtained of the different samples according to the invention of encapsulated retinal (graph below).

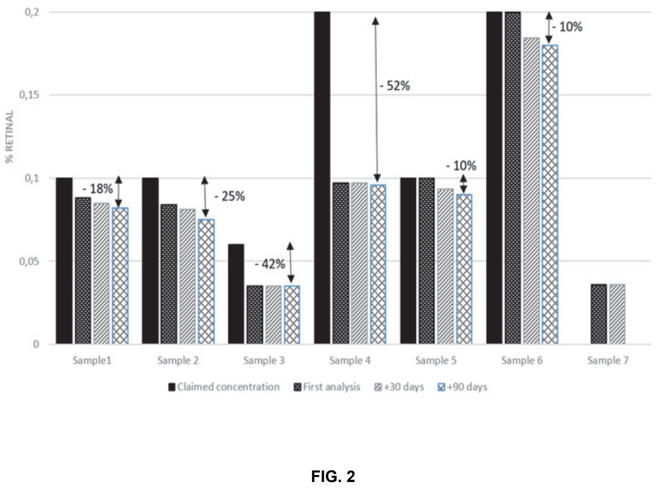

FIG. 2 is a comparative graph showing the stability of retinal in different compositions according to the invention and in comparative samples, as determined by reverse-HPLC with 350 nm wavelength detection at room temperature.

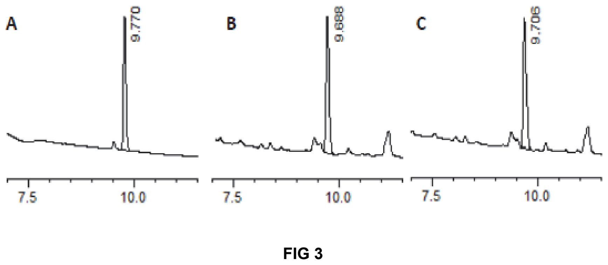

FIG. 3 depicts three HPLC chromatograms. One for Acetyl tetrapetide-2 (FIG. 3A), another for the cosmetic composition according to the present disclosure with retinal at 0,1% w/w just after preparation (time 0) (FIG. 3B), and another one for the cosmetic composition according to the present disclosure with retinal at 0,1% w/w after being kept for three months at room temperature (FIG. 3C).

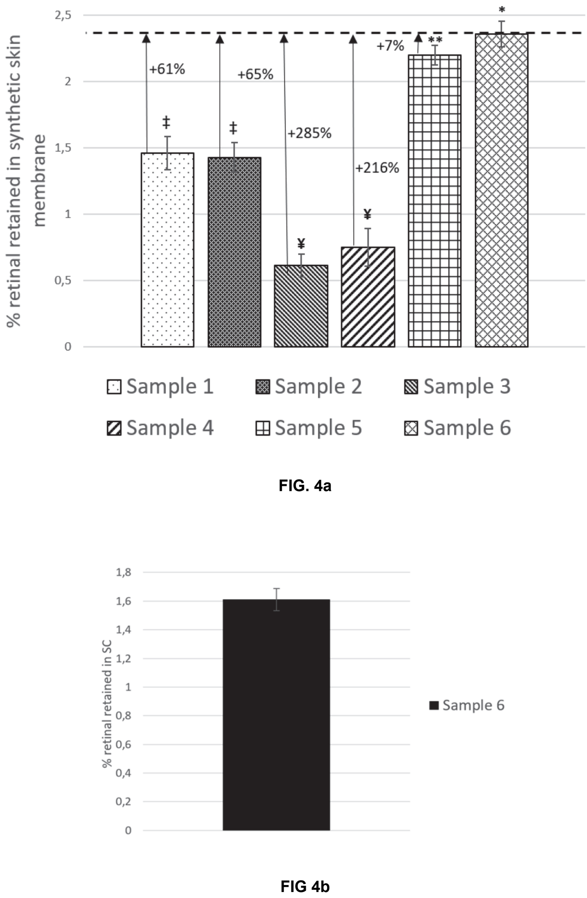

FIG. 4. FilG 4a is a graph depicting the amount of retinal absorbed in synthetic skin, when using the compositions according to the present disclosure in two retinal concentrations and the amount of retinal absorbed in comparative compositions. FIG. 4b is a graph showing the penetration of the composition according to the invention having a retinal concentration of 0.2w/w%, according to the in vivo tape stripping method.

### DESCRIPTION OF THE DISCLOSURE

The foregoing and other advantages and features will be more fully understood from the following detailed description of the invention. The examples disclosed herein are to be taken in an illustrative and not limitative way.

Unless otherwise indicated, all percentages relating to the content of a component or to a collection of components of the composition of the present invention refer to the weight percentage with respect to the total weight of the composition.

In the context of the present disclosure, the formulations "according to the disclosure", "preparation according to the disclosure", etc. always refer to the preparations, processes and uses according to the disclosure, i.e. also to preparations in which the uses according to the disclosure are realized as well as preparations with which the inventive method is realized.

Unless the context clearly requires otherwise, throughout the description and the claims, the words "comprise", "comprising", and the like are to be construed in an inclusive sense that is to say, in the sense of "including, but not limited to".

The cosmetic composition is as defined in the present disclosure, that is, a cosmetic composition for rejuvenating the skin comprising from 0.01% to 0.5% by weight with respect to the total weight of the composition of a precursor of retinoic acid selected from the group consisting of retinaldehyde, retinol, retinyl retinoate, retinyl esters and combinations thereof, from 0.0001% to 0.01% by weight with respect to the total weight of the composition of an anti-aging or antioxidant peptide, and from 0.0000005% to 0.000005% by weight with respect to the total weight of the composition of a growth factor, wherein the precursor of retinoic acid is encapsulated by an encapsulating agent comprising phospholipids. The compositions of the invention allowed highly effective compositions, providing wrinkle reduction, good skin luminous, brightness, firmness and elasticity, with very low peptide concentrations.

Preferably, the precursor of retinoic acid is retinaldehyde. Retinaldehyde and retinal are equivalent terms. Therefore, in the present disclosure, retinal and retinaldehyde may be equivalently used and refer to the compound having a C₂₀H₂₈O as general formula, a molecular weight of 284,214 g/mol and a CAS number 311338-94-0. In a preferred embodiment, the composition comprises from 0.02% to 0.4% or from 0.1% to 0.2% by weight of retinal with respect to the total weight of the composition. The compositions of the invention comprising retinal allow highly effective compositions, which provide good wrinkle reduction, good, improved skin luminosity and brightness, improved firmness and elasticity, while containing very low amounts of the active, retinal. The compositions of the invention comprising retinal also proved to be suitable for all skin types (dry, oil, normal and combination skin) and did not show any intolerance when applied on the skin, such as irritation, burn, redness or itching, in any of the subjects.

Furthermore, the compositions of the invention comprising retinal, as the retinoic precursor, showed a reduction of the spots on the skin of the tested subjects in 70 % as well as a better appearance of the pores of the skin in 87% of the tested subjects.

An anti-aging peptide or antioxidant peptide is a small chain of aminoacids, a peptide, that plays a role in counteracting the biological effects of aging, often by neutralizing free radicals or protecting cells from oxidative stress. These peptides may work in various ways to support skin health. In particular, the composition of the invention comprising anti-aging peptides as described herein, showed improved appearance of the skin, reduced wrinkles, enhancing cellular function, and promoting tissue repair, protecting the cells from damage caused by free radicals-unstable molecules that can cause oxidative stress, leading to cell damage and aging, neutralizing free radicals or modulating antioxidant pathways within the body to reduce oxidative damage.

Preferably, the anti-aging or antioxidant peptide, present in the composition of the invention, is selected from the group consisting of carnosine, copper tripeptide-1, pentapeptide-18, palmitoyl tetrapeptide-7, palmitoyl pentapeptide-4, nonapeptide-1, hexapeptide-2, acetyl hexapeptide-8, acetyl octapeptide-3, acetyl hexapeptide-1 o acetyl tetrapeptide-2, and combinations thereof. Thus, these compositions showed very good efficacy using low antioxidant peptide concentrations (by weight of the total composition), showing good absorption on the skin and significant skin volume wrinkle reduction, which decreased down to 52 % (also referred as - 52%) after 28 days. Furthermore, the skin showed improved luminosity and brightness up to 4%, after 28 days of the application of these compositions in all volunteers (male and female). As well as very good elasticity and firmness on the skin.

In a preferred embodiment, the anti-aging or antioxidant peptide is not acetyl tetrapeptide-11 or the skin composition of the invention is free from acetyl tetrapeptide-11, as when used in the composition of the invention it might cause mild side effects like skin irritation or occasionally allergic reaction.

Even more preferably, the anti-aging or antioxidant peptide used in the composition of the invention is acetyl tetrapeptide-2, which provided compositions which showed good antioxidant activity inhibiting the aryl receptor (AHR) and stimulating the NRF2-like Nuclear Factor (erythroid-derived factor 2) that activates the detoxifying and endogenous antioxidant systems HMOX-1, GSS, GPX1, GSTP1 and TRX1. Additionally, when the composition comprises acetyl tetrapeptide-2, the composition showed an improved brightness and skin luminosity, as well as good stability over time.

Preferably, the cosmetic composition as disclosed herein comprises an anti-aging or antioxidant peptide in an amount of from 0.0005% to 0.005% by weight with respect to the total weight of the composition, more preferably from 0.00075 to 0.0025, more preferably about 0.001%. Furthermore, the anti-aging or antioxidant peptide ranges used allow the formation of stable liposomes and the use of higher amounts of phospholipids and liposomes, providing high retinal loading efficiency and better stability over time even when stored at room temperature.

A growth factor is a substance, usually a protein or a steroid, that stimulates cell growth proliferation, healing, and differentiation. Growth factors play a crucial role in regulating a variety of cellular processes, including embryonic development, tissue repair, immune responses, and the maintenance of normal cellular functions. They act as signalling molecules that bind to specific receptors on the surface of cells, triggering intracellular pathways that promote various cellular activities. Preferably, the cosmetic composition of the invention comprises a growth factor as disclosed herein, which is selected from the group consisting of epidermal growth factor, also known as Oligopeptide-1, insulin-like growth factor 1, transforming growth factor beta-2, Nicotiana Benthamiana Hexapeptide-40 sh-Polypeptide-76, sh-Polypeptide-1, sh-Polypeptide-9, sh-Polypeptide-11, Oligopeptide-4, Oligopeptide-2, and combinations thereof. More preferably, cosmetic compositions of the invention comprise the growth factor, Oligopeptide-1, also known as epidermal growth factor (EGF), and/or combinations thereof. Said cosmetic composition showed an increase of the keratinocyte's growth.

The epidermal growth factor (EGF) is preferably obtained using Nicothiana Benthamiana as plant biofactory, since glycosylations and a tertiary structure similar to endogenous growth factor may be obtained. These compositions provide cosmetic compositions with high efficacy.

EGF is a widely known therapeutic polypeptide that exerts a mitogenic effect by binding to its membrane receptor EGFR. The compositions of the invention, comprising EGF, showed fibroblasts stimulation and increased the synthesis of collagen and hyaluronic acid. Additionally, improvements in skin roughness, by improving the elasticity up to 46 % (elasticity over the entire surface, R7) and reduction of wrinkles volume down to 52 % (-52 %) and the area of the wrinkles down to 51 % (-51 %), were also observed.

Preferably, the cosmetic composition of the invention comprises the growth factor in an amount from 0.000001% to 0.000003% by weight with respect to the total weight of the composition, as this range showed an improvement in the efficacy, such as elasticity and firmness as well as good antiaging effect of the retinoic acid precursor, as it was shown for retinal. More preferably the amount of growth factor is from 0,0000015 to 0,0000025, even more preferably from 0.0000017 to 0.0000023. Compositions comprising less than 0.000001% showed less efficacy of the composition on the skin. Working with higher amounts above 0.000003%, may cause toxic buildup and does not provide better efficacy of the composition of the invention, it may also promote the formation of spots on the skin.

A liposome is a spherical structure composed mainly of a lipid bilayer, surrounding an aqueous core which can be used in cosmetics as delivery systems. Liposomes usually have a particle size from 20 nm to 5 µm and may be obtained from a wide variety of lipids, such as phospholipids. A phospholipid has a polar or hydrophilic group at the head and two hydrophobic hydrocarbon tails. When such compounds are in the presence of excess aqueous solution, they organize themselves in a way that minimizes interactions between water and hydrocarbon chains. This system triggers the spontaneous formation of lipid lamellae in the form of spherical bilayers. The lamellar structure of liposomes may allow the encapsulation of hydrophilic molecules in their aqueous core and hydrophobic molecules inside their lipid bilayer.

In the cosmetic composition as disclosed herein, the phospholipids are preferably derived from lecithin. Lecithin is usually the membrane component of the liposome. Lecithin may be obtained from vegetable products such as soybeans, peanuts, cottonseed, sunflower, rapeseed, corn, or ground nut oils. Lecithin can also be obtained from eggs. Lecithin comprises mixtures of glycerophospholipids including phosphatidylcholine, phosphatidylethanolamine, phosphatidylinositol, phosphatidylserine, and phosphatidic acid. So, the phospholipids according to the present disclosure may be selected from the group consisting of phosphatidylcholine, phosphatidylethanolamine, phosphatidylinositol, phosphatidylserine, phosphatidic acid, and combinations thereof. Lecithin comprises phospholipids, which can present problems connected with their physicochemical stability (due to the unsaturated chains, which are susceptible to air oxidation). Lecithin may contain from 25-80 % of the phospholipid, phosphatidylcholines (PCs), and even up to 98 %. The composition of the invention comprises phospholipids derived from lecithin, wherein the lecithin comprises phosphatidylcholines in an amount by weight of about 60% to 90%, to the total weight of the lecithin, preferably from 65% to 85 %, or from 70% to 80%, or 65% to 75%, as these ranges provide a stable cosmetic composition Suitable lecithin may be a lecithin derived from soy.

Thus, the compositions of the invention and the combination of the elements present in the composition provide the advantages that said compositions are stable after manufacturing and during storage avoiding the degradation of the active compounds, as for example, the retinoic acid precursors, preferably avoiding the degradation of retinal, as well as ensuring the stability of the excipients, as shown in table 1.

Preferably, in the cosmetic composition according to the present invention the phospholipids are present in an amount of from 0,1% and 2% by weight with respect to the total weight of the composition. Preferably, the amount of phospholipids is from 0,3% and 1%, more preferably from 0,4% and 0,8%, even more preferably from 0.5 to 0.7%. The molar ratio of phospholipid: retinoic acid precursor, preferably retinaldehyde, is from 15:1 to 5:1, more preferably from 10:1 to 8:1, as also contributes to improve the stability of the retinoic acid precursor, preferably to improve the stability of the retinal.

In a preferred embodiment, the encapsulating agent, in the cosmetic composition, may be a liposome. Preferably, the liposomes used may comprise one lipidic bilayer, two or more lipidic bilayers, or mixtures thereof.

The cosmetic composition according to the present disclosure may further comprise an emollient.

An emollient in cosmetics is a substance that softens and smooths the skin by forming a protective barrier on the surface. It helps to maintain moisture in the skin, preventing dryness and irritation. The compositions of the invention make the skin feel softer, more hydrated, and less rough or flaky, when comprising at least one emollient. The emollients in the cosmetic composition according to the present disclosure may preferably be selected from the group consisting of squalene, sweet almond oil and combinations thereof.

The cosmetic composition according to the present disclosure may also comprise water and aliphatic polyalcohol, the aliphatic polyalcohol being preferably selected from the group consisting of glycerol, butylene glycol, caprylyl glycol and combinations thereof.

The composition of the invention may be prepared by a process that comprises the steps of:
1. preparing a liposome raw material solution containing at least one retinoic acid precursor, preferably retinal, water and phospholipids, wherein the retinoic acid precursor is encapsuled in the phospholipid,
2. adding at least a growth factor and at least an oligo peptide to the product obtained in the previous step 1, followed by stirring at a temperature from 25 to 90 °C until a homogeneous mixture is obtained,
3. decreasing the temperature of the mixture obtained in the previous step to room temperature, and
   more preferably, the retinoic precursor used in step 1 consists of retinal.
4. Optionally, adjusting the pH of the mixture obtained in step 3 to a range from 5-6.5

In a preferred embodiment of the process to prepare the cosmetic composition of the invention, the liposome raw material solution of step 1 is prepared by a process which comprises the steps of:
1.1 Preparing an aqueous phase by mixing water and cosmetic acceptable excipients followed by stirring at a temperature from 60 up to 100 °C
1.2 Adding a lipid phase over the aqueous phase obtained in step 1.1,
   - wherein the lipidic phase is obtained by mixing at least an organic solvent and a phospholipid, at a temperature from 60 up to 100 °C, followed by stirring, until an emulsion is obtained,
   - and wherein the addition of the lipid phase to the water phase is carried out at a temperature from 60 up to 100 °C,
1.3 Decreasing the temperature of the mixture obtained in step 1.2 until it reaches a temperature range from 30-50 °C, followed by addition of a retinoic acid precursor, preferably retinal, while stirring, and
1.4 Optionally, adjusting the pH of the mixture obtained in step 1.3 to a range from 5-7.

The pH of the mixture generated in step 1.4 is preferably adjusted to a pH range from 5.5 to 6.5, to provide a more adequate viscosity for skin applications.

The retinoic acid precursor, such as retinal, may be added in step 1.3 as a premix, wherein the premix was prepared my mixing and stirring at least the retinoic acid precursor, water and lipids, such as phospholipids, at a temperature range from 15-35 °C, preferably from 20 to 30 °C.

The excipient used in step 1.1 is and excipient suitable for an aqueous phase, such as humectants selected from the group consisting of glycerine, propylene glycol, butylene glycol and pentylene glycol, or viscosity modifiers selected from the group consisting of hydroxyethylcellulose, xanthan gum, carbomer and acrylates/C10-30 alkyl acrylate crosspolymer or preservatives selected from the group consisting of, phenoxyethanol, ethylhexylglycerin, caprylyl glycol, sodium benzoate, potassium sorbate, benzyl alcohol and 1,2-hexanediol.

In a preferred embodiment of the process to prepare the cosmetic composition of the invention, the composition of the invention is an emulsion.

In another preferred embodiment of the process to prepare the cosmetic composition of the invention, wherein the retinoic acid precursor is retinal, and the phospholipid added is lecithin.

As described herein, the present disclosure also relates to a method for rejuvenating the skin, the method comprising topical application of the cosmetic composition as disclosed herein, preferably, wherein the retinoic acid precursor is retinal.

As described herein, the present invention also relates to the use of the cosmetic composition according to the present disclosure for rejuvenating the skin by means of topical application, preferably, wherein the retinoic acid precursor is retinal, more preferably the composition is a cream or serum composition.

The cosmetic composition described herein comprising retinaldehyde, as the retinoid acid precursor, allowed a stable formulation over time up to over 3 months, and even until 6 months after opening, good skin penetration of the cosmetic composition on the skin, high anti-wrinkle efficacy, reduction of spots, better appearance of pores, as well as providing the skin with good luminosity effect and with good values of elasticity/firmness. Said composition was suitable for both genders, male and female and for all type of skins, normal, combination, dry and oil skin and did not cause any causing undesirable side effects such as dryness or skin irritation.

### EXAMPLES

Retinal is a highly unstable molecule to light, heat, oxygen and free radicals. In addition, it is a fat-soluble ingredient, which makes it difficult to incorporate into aqueous formulations. In an example of the present invention, the cosmetic composition contains retinal as, the retinoic acid precursor. Retinal was encapsulated as described in example 1. The obtained composition showed improved solubility, stability and absorption into the skin.

### Example 1: Retinal encapsulation preparation.

Pure retinal was encapsulated in liposomes using a lipid matrix composed of phosphatidylcholine and other lipids from non-GMO soybeans.

Firstly, water and excipients suitable for the aqueous phase were added separately, one by one to a reactor followed by vigorous stirring at a temperature above 70 °C, to provide a first phase (phase A).

Secondly, in a separate container, an oil phase comprising the lipid matrix containing phosphatidylcholine and other lipids from non-GMO soybeans were added and mixed while stirred at a temperature above 70 °C, to form a second phase (Phase B).

A lipid matrix is composed of phosphatidylcholine and was obtained from lecithin. A lipid matrix, as known in the common general knowledge, is designed to mimic the natural protective barrier of the skin or to transport active ingredients. Excipients suitable for the aqueous phase are generally glycerin, propylene glycol, butylene glycol, pentylene glycol, hydroxyethylcellulose, xanthan gum, carbomer, acrylates/C10-30 Alkyl acrylate crosspolymer., phenoxyethanol, ethylhexylglycerin, caprylyl glycol, sodium benzoate, potassium sorbate, benzyl alcohol, 1,2-hexanediol.

Once the previous steps are completed, both phases A and B, were mixed in the reactor at a temperature of 70 °C by adding phase B over phase while stirring, until an emulsion was formed. Afterwards, the emulsion was cooled down to 35-45 °C and a retinal premix was added to the reactor while stirring. The retinal premix was prepared my mixing the retinaldehyde, water, and phospholipids, at a temperature range from 20 to 30 °C.

If necessary, the pH of the final mixture was adjusted to reach a range from 5-6.5. The pH of the composition of the invention may be adjusted using for example a diluted solution of citric acid, NaOH, lactic acid, gluconic acid, arginine or any citrate, gluconate or lactate of alkali metals or alkaline earth metals, until the desired pH is reached.

The composition obtained with encapsulated retinal was analysed by Dynamic Light Scattering, a technique which allows to measure the particle size and the Z potential of vesicles formed.

More specifically, by using a type of Dynamic Light Scattering, such as electrophoretic light scattering (ELS), a technique where charged particles are moved through a liquid by an electric field and their velocity is measured. This parameter is a measurement of the magnitude of the electrostatic repulsion or attraction between particles, a relevant parameter to determine the stability of a formulation composed of liposomes. The Z-potential is the electric potential in the interfacial double layer (DL) at the location of the slipping plane relative to a point in the bulk fluid away from the interface. In other words, zeta potential is the potential difference between the dispersion medium and the stationary layer of fluid attached to the dispersed particle.

Systems which show values above approximately ±30 mV are considered highly stable, as the high surface charge causes particles to repel each other strongly.

Systems which show values between and ±$10$ mV are considered unstable, as these values indicate limited stability and a high likelihood of coagulation or flocculation.

The sign (+ or -) indicates the surface charge, while the magnitude is the primary factor for stability.

The liposomes encapsulating retinal showed a Z potential lower than -60 mV and an approximate size of 0,35 microns of diameter, as shown in FIG. 1. The formed liposomes form with retinal are not nano retinal liposomes. Thus, in this case the formed emulsion of example 1 provided a highly stable system, having a Z potential of -60 mV. The process is very reproducible and reliable providing systems with high Z potential as seen in FIG. 1.

### Example 2: Formulation of the cosmetic composition

The cosmetic composition according to the present invention was prepared from the obtained encapsulated retinal composition, as described above (example 1). Two different cosmetic compositions, having 0.1% or 0.2% of retinal (by weight with respect to the total amount of the compositions) were prepared. Two other active ingredients were added to the composition and mixed by stirring, so, as to provide a retinal composition:
- Epidermal Growth Factor (EGF) (≥95.0% purity), also known as Oligopeptide-1, was added so that EGF was in an amount of 0,0000021% by weight with respect to the total amount of the cosmetic composition.
- Acetyl tetrapeptide-2 (> 95% purity) was added so that Acetyltetrapeptide-2 was in an amount of 0,001% by weight with respect to the total amount of the cosmetic composition.

If necessary, the pH of the final mixture was adjusted to reach a range from 5-6.5. The pH may be adjusted using for example a diluted solution of citric acid, NaOH, lactic acid, gluconic acid, arginine or any citrate, gluconate or lactate of alkali metals or alkaline earth metals, until the desired pH is reached.

### Example 3: Stability studies

Due to its five conjugated double bonds, retinal is a highly unstable molecule to light, heat, oxygen and free radicals, prone to addition, oxidation, polymerization, cleavage and/or dehydration reactions. For this reason, it is of vital importance to study the stability of the active ingredient in the composition and in respect of rest of the ingredients, also referred as compatibility studies. This study was carried out by means of HPLC. The amount of the retinal present in the cosmetic composition of example 2 was quantified. Two different compositions according to the invention were prepared, having two different retinal concentrations by weight to the total weight of the compositions, 0.2% and 0.1%, referred as Retinal Renewal 0,1% and Retinal Renewal 0,2% (also named in this application as, sample 5 and sample 6 respectively, from manufacturer (C)). The amount of retinal was measured after 30 days (1 month) and after 90 days (3 months) in stability.

The stability of these two compositions was compared to other retinal formulations commercially available. The tested compositions are summarized below (Table 1):

**Table 1: Stability of compositions of the inventions and comparative compositions comprising retinal.**

| Sample | Manufacturer | Retinol precursors | Characteristics of the Retinal | Other components | Retinal concentration as described in label. % w/w) | Retinal concentration at 30 days (% w/w) | Retinal concentration at 90 days (% w/w) | Retinal weight variation at 90 days (%) |
|---|---|---|---|---|---|---|---|---|
| 1 | A | Retinal, Sodium, Retinoyl hyaluronate | Not Encapsulated | No Growth Factor, No Antioxidant Peptides | 0.1 | 0.085 | 0.082 | 18% |
| 2 | A | Retinal, Retinol, sodium Retinoyl hyaluronate | With Liposomes Phospholipids | No Growth Factor, No Antioxidant Peptides | 0.1 | 0.081 | 0.075 | 25% |
| 3 | B | Retinal | Crystal Encapsulation | No Growth actor, No Antioxidant Peptides | 0.06 | 0.035 | 0.035 | 42% |
| 4 | B | Retinal | Crystal Encapsulation | No Growth Factor, No Antioxidant Peptides | 0.2 | 0.097 | 0.096 | 52% |
| 5 | C | Retinal | Encapsulated ising liposomes | Growth Factor, Antioxidant Peptides | 0.1 | 0.093 | 0.090 | 10% |
| 6 | C | Retinal | Encapsulated using liposomes | Growth Factor, Antioxidant Peptides | 0.2 | 0.184 | 0.180 | 10% |
| 7 | D | Retinal, Retinol, Retinyl Propionate | "Your Liposomal mist" | Growth Factor NO Antioxidant Peptide | | 0.036 | | |

Sample compositions 1 and 2 correspond to the same manufacturer (A) having both 0,1 w/w% of retinal. Composition sample 1 has non-encapsulated retinal and composition sample 2 has liposomal retinal. Neither compositions, 1 or 2 comprise Growth Factor nor Antioxidant Peptides.

Sample compositions 3 and 4 correspond to manufacturer B and contained encapsulated (crystal encapsulation) retinal having a concentration of retinal of 0.06 and 0.2w/w% respectively and neither of them comprise Growth Factor nor Antioxidant Peptides.

Sample compositions 5 and 6 correspond to those of example 2 of the present application (retinal renewal 0.1 and retinal renewal 0.2% respectively), comprising encapsulated retinal with phospholipids, wherein the concentration of retinal is 0.1 and 0.2 w/w% respectively. Both contain at least one growth factor and one antiaging peptide.

Sample composition 7 corresponds to the commercial product cited in the prior art internet anonymous publication entitled "Your Liposomal mist", as previously described. Said product was acquired and tested. The internet anonymous publication "Your Liposomal mist", disclosed that said commercial composition comprised a mixture of different encapsulated retinols, including, retinol, retinal and retinyl propionate. Said internet publication did not describe the amount or w/w% of the retinal or retinoids added in the commercia composition.

To carry out the stability studies, all samples were stored in closed glass containers (close dish) and evaluated at different time intervals (e.g., time 0, 1 month, 3 months, etc.) and stored at ambient conditions (20 °C). The analyses performed at times mentioned above were compared to the baseline values to identify possible significant deviations. The commercial compositions were acquired as close as possible to the date of manufacture.

Quantification of retinol/retinal was performed by high performance liquid chromatography (a Shimadzu Series 20 Prominence instrument), by means of an internal validated method as described herein. The HPLC instrument comprised one solvent delivery pump LC-20AT, a SIL-20^{a} HT automatic injector, a CBM-20A controller, and a UV-VIS detector SPD-M20A (at 220 nm). The column used was an Aeris PEPTIDE XB-C18 100 Å reversed-phase column (4.6 x 250 mm) of 5 µm particle diameter. Column temperature: 40 ° C. The samples were eluted at a flow rate of 1 mL·mir⁻¹ using a mobile phase having a lineal gradient of 0.045 % TFA/H2O (A) and 0.036 % TFA/ACN (B) injection volume 5.0 µL,

The retinaldehyde standard solution was prepared by first weighing 0.01 g of retinol and measuring up to 100 mL of FTA (100 ppm). Then, this was diluted to 10 ppm, 5 ppm, and 1 ppm, and these three points were used as calibration curves. For liposomes, 5 µL of the liposome solution immediately after the heating preparation was collected, and 995 µL of ACN (acetonitrile) was added and dissolved to prepare a measurement sample (diluted 200 times). Results

It was observed that within the first 3 months the compositions of the invention, composition sample 5 and 6, were the ones showing less degradation, less than 10% (see FIG. 2). Neither of the two samples 5 and 6 (Retinal renewal 0,1% or Retinal renewal 0,2% respectively) according to the present cosmetic composition showed significant degradation. For these products the degradation occurred mainly during the first month of the product's life, since during the manufacturing process the ingredient was more exposed to external conditions such as the presence of oxygen or light. After the first month, the concentration remained practically constant over time. The compositions of the invention remained in optimal condition throughout the entire life cycle of the product with degradation values similar to those obtained after 3 months.

The stability of the retinal in the comparative compositions (competitor products) versus the compositions of the invention were also analysed. In this case it must be considered that the period of time elapsed between the production of the product by the manufacturer and the date on which the product was purchased and analysed was reduced as much as possible, requesting new batches. In none of the products analysed was the concentration claimed by the manufacturer found, as shown in table 1. It should be noted that at the time of the analysis these products had been recently manufactured, and they had just been launched on the market.

In the case of product compositions 1 a non-encapsulated without a growth factor or antioxidant peptides, the stability results showed a decrease of the active retinal after 1 month of around 15% after 1 month and 19% after 3 months. Contrary, composition 2 (liposomal composition) showed worse stability results, as a decrease of the active retinal to 19% was observed after 1 month and down to 25% after 3 months (difference between the concentration claimed on the products and the concentration measured).

In the case of composition samples 3 and 4, these suffered a greater degradation of the active retinal. For example, in the case of commercial sample 3 the initial concentration of retinal (0.06 %) dropped 42% after 1 month and in the case of composition sample 4 (0.2%) the retinal concentration plummeted to 51 %. Thus, only 0.097% of initial retinal remained in the commercial product.

Sample 7, which corresponded to the commercial product cited in the prior art internet anonymous publication entitled "Your Liposomal mist", as previously described", also showed a very low amount of retinal after 1 month, with amounts of retinal of 0.036 w/w%, very similar to the results obtained for sample 3.

Other components, as the Acetyl tetrapeptide-2, which may be susceptible to degrading in composition containing retinal, proved to be stable in the composition of the invention, when in contact with retinal and other ingredients of the cosmetic composition. As seen in analysis by HPLC which showed that the retention time and the area under the curve for the peak corresponding to Acetyl tetrapeptide-2 in the control and in the cosmetic composition at time 0 and after three months was equivalent.

### Example 4: Efficacy studies

### Percutaneous absorption

Percutaneous absorption studies are widely used in the pharmaceutical, cosmetic and dermatological industries to evaluate the efficacy and safety of topical products, as well as to better understand the mechanisms of skin absorption. These studies are performed using a device called a Franz cell, which consists of two compartments separated by a membrane. In the upper compartment the substance to be studied is placed, while in the lower compartment a receptor medium that simulates the pH, salinity and temperature conditions under the skin is placed. The membrane used in the Franz cell is key in simulating the skin barrier and measuring the absorption of the substance through it. This membrane can be of animal or artificial origin, made of different materials, such as cellulose or synthetic polymers. In our case we used a commercial synthetic membrane called Strat-M^{®}, which has a multilayer structure that mimics the composition and characteristics of the skin. This membrane is very useful in basic and applied research studies, as it reduces the need to use human or animal skin in experiments, which have ethical and regulatory advantages. In addition, it has been widely demonstrated to provide consistent, reproducible results with a high correlation to those obtained in human skin.

During the study, the integrity of the skin barrier and the tightness of the experimental model are verified for each diffusion cell before the application of the cosmetic products by measuring the trans-epidermal water loss (TEWL). The sample to be analysed is then applied to the donor compartment and left in contact with the synthetic skin for 24 hours. After the contact time has elapsed, the three parts of the cell (donor, membrane and receptor) are sampled separately. Finally, the percutaneous absorption of the active ingredient is studied quantitatively by HPLC. Each sample was analysed in triplicate and on two different days to avoid human error and environmental effects. The average of these measurements was calculated; their standard deviation and the statistical significance of the data was analysed by Student's t-test.

FIG. 4a shows the results of percutaneous absorption of retinal in the different products tested. The efficacy of the compositions was evaluated by studying the cosmetic composition according to the disclosure in its two concentrations sample 5 and sample 6 (Retinal Renewal 0,1% and Retinal Renewal 0,2% respectively) and its analogues (Comparative compositions 1, 2, 3 and 4).

It was found that for both concentration, sample 5 and sample 6 had a high absorption into the skin (see FIG. 4a). These compositions according to the disclosure were also tested and compared with several competitor products (compositions 1-4, as described in table 1), proving that the samples of the invention improved retinal penetration in the skin in all cases.

It is worth noting the large differences found with sample 3 (0.06%), wherein a significant drop in penetration of -285% occurred.

Likewise, sample 4 (0.2%) showed a sharp drop down to -216% when compared to the composition of the invention, sample 6 (0.2%).

The of the invention, as seen in samples 5 and 6, showed always better penetration in the skin as compared to sample compositions 1 or 2. For composition sample 6 (0.2%), a penetration improvement of +61% was observed compared to composition sample 1 (0.1%). Similarly, composition sample 6 (0.2%) showed a 65% improvement in penetration compared to composition sample 2 (0.1%). In the case of composition sample 5 (0.1%), a 50% penetration improvement was observed compared to composition sample 1 (0.1%), and a 54% improvement compared to composition sample 2 (0.1%).

### In vivo tape stripping

In order to validate the previously *in vitro* percutaneous absorption results, an *in vivo* study known as tape stripping was performed. This is a technique that involves the application of a special adhesive tape on the skin, followed by its removal to remove superficial layers to quantify the amount of active ingredient that has been retained in the stratum corneum of the skin.

To do this, the area of the volunteer's forearm (approximately 3.8 cm²) is first cleaned and dried to ensure that there are no contaminants that could affect the results. A standardized amount of product sample is then applied to the area, massaged to facilitate absorption and allowed to stand for two hours. After this contact time, the area is cleaned again with abundant water. To remove the most superficial layer of the skin, medical adhesive tape (Standard D-Squame^{®} Discs (CuDerm Corporation) is used, which is pressed firmly on the skin to ensure good adherence and is quickly removed, taking with it a layer of stratum corneum cells. This process is repeated twenty times at the same site to remove successive layers of skin. Finally, the removed strips (diameter: 22 mm) are treated with an extractant solution, which is then analysed to quantify the amount of substance that penetrated the skin by HPLC.

HPLC Method: Analytical RP-HPLC was performed on a Shimadzu Series 20 Prominence instrument comprising one solvent delivery pump LC-20AT, a SIL-20^{a} HT automatic injector, a CBM-20A controller, and a UV-VIS detector SPD-M20A. The column used was an Aeris PEPTIDE XB-C18 100 Å reversed-phase column (4.6 x 250 mm) of 5 µm particle diameter. The samples were eluted at a flow rate of 1 mL·min-1 using lineal gradients of 0.045 % TFA/H2O (A) and 0.036 % TFA/ACN (B) and UV detection at 220 nm. This study was performed with the composition defined herein having a retinal concentration of 0.2% w/w (sample 6). The results obtained (see FIG. 4b) are in agreement with those obtained in the in vitro study, showing that the compositions of the invention have a good penetration in the skin.

### Example 5: Anti-wrinkle efficacy

A single-centre open-label study with dermatological and instrumental evaluation was performed for 28 days with 30 volunteers of both genders, men and women, having ages comprised from 30 to 70, having normal skin and instructed not to use any cosmetic products in the area to be evaluated that might alter the results of the trial in a week prior to the study. The anti-wrinkle efficacy was quantitatively evaluated, by means of instrumental measurements of the skin of the face for which the Visioface 1000D^{®} equipment was used, taking measurements on the volunteers before the use of the tested product, after 14 days and after 28 days of use of the tested product.

The measurements were carried out on the application area (in different areas of the face), verifying the volume, area and depth of the chosen wrinkle. Measurements were taken before the application of the product (T0), after 14 days (T14D) and after 28 days (T28D) of regular use. The study was carried out in a heated room with an ambient temperature of 20 ± 2°C and a relative humidity of 45 ± 15%. The efficacy of the product (shown in table 2) is confirmed if positive results are obtained in more than 50% of the subjects. These were the results achieved in this study.

The results are summarized in the following table (Table 2):

**Table 2: Mean results obtained regarding the measurements of depth, width and volume achieved after comparing the measures performed after the application of the product (T0), after 14 days (T14D) and after 28 days of the use of the product (T28D).**

| | T0 | T14D | T28D | T0-T14D | T0-T28D |
|---|---|---|---|---|---|
| VOLUME | 133806,800 | 98354,000 | 68908,667 | -30% | -52% |
| AREA | 10895,967 | 7573,500 | 5636,033 | -32% | -51% |
| DEPTH | 11,233 | 11,667 | 10,900 | 4% | -2% |
| % AREA | 1,411 | 0,974 | 0,726 | -32% | -51% |

Table 2 shows that the volume, area and depth of the wrinkles of samples tested (samples prepared in example 2, retinol renewal 0.1% and retinol renewal 0.2 %) were significantly decreased after 14 and 28 days of application of the product. It is concluded that said composition has an anti-wrinkle effect as it reduces significantly, the depth, width and volume of wrinkles after 14 days and even more after 28 days, where the volume (mean value of -52%), the area (mean value of -51%) and the depth (mean value of -2%) of the wrinkles with a reduction of the area of -51%.

Furthermore, the compositions of the present invention exhibit superior anti-wrinkle effects when compared to prior art compositions. For example, the antioxidant cream disclosed the anonymous internet publication entitle "3X Antioxidant Cream" (as described in the prior art section) resulted in a modest anti-wrinkle effect after two weeks of use, with a 15.9% improvement in deep wrinkles, a 12.7% improvement in fine wrinkles, and a 22.7% improvement in induced wrinkles. These values were significantly lower than the results obtained with the composition of the present invention which provided improvements of 51 and 52 %

In the case of the anonymous internet publication "Retinol Super Bounce Serum", the serum composition described therein, claimed that said serum provides an anti-wrinkle effect when used on the skin. Said effect seems low, with -10.6% wrinkles reduction lips, -8.5% wrinkles reductions in the smile line or wrinkles around the eyes of -7.0%. All these values were significantly lower than those provided by the composition of the invention (with 51 ad 52 % wrinkle reduction, as shown in table 2), even though the amount of retinol precursors added to the "Retinol Super Bounce Serum" were significantly than in the composition of the invention.

### Example 6: Luminosity effect

The same single-centre open-label study with dermatological and instrumental evaluation as described for Example 5 was used to measure luminosity effect. For the instrumental evaluation of the efficacy on brightness with the Chromameter CR-400, one of the readings was taken on all volunteers before the application of the product at the sites established at the baseline visit (front). Three measurements were taken at each time/area 14 days and 28 days after the start of the study.

Where the individual results were expressed in absolute values for each time and in percentage variation between times T0-T14D and T0-T28D. Measuring Principle of Chromameter CR-400. The Skin-Colorimeter probe has a large illumination area, so that sufficient light reaches the surface of the skin for measurement, but a small enough measurement area does not ensure that the surface colour is measured and not the colour of the deeper layers of the skin.

The probe sends out white LED light, arranged circularly to illuminate the skin evenly. The emitted light is scattered in all directions, some of it travels through the layers and some is scattered away from the skin, the light reflected from the skin is measured by the probe and expressed accordingly.

The raw data of the probe is corrected with a special colour matrix to be as close as possible to normal values. Due to the unique structure of the skin and the light source of the probe, the skin cannot be measured according to standards such as DIN (the German industry standard) or others. The measured values are close to those standards but are shown as arbitrary results. The measured skin colour is expressed as an XYZ-value (tristimulus) and can be calculated in different colour spaces.

### L*a*b:

The CIELAB colour space (also known as CIE L*a*b*) is a colour space defined by the International Commission on Illumination (CIE) in 1976. It expresses colour as three values: L* for lightness from black (0) to white (100), a* from green (-) to red (+), and b* from blue (-) to yellow (+). CIELAB was designed so that the same amount of numerical change in these values corresponds to approximately the same amount of visually perceived change. It is a concept that is often used to characterise skin.

Correlation between colour space and human skin:
- L* gives information on the black-white axis, brightness/lightness, the higher the L*, the brighter the skin.
- The a* value on the green-red axis is proportional to the erythema (microcirculation/redness of the skin).

A higher a* value corresponds to a higher erythema.
- The b* value on the blue-yellow axis in the literature often describes pigmentation.

The instrumental evaluation of the efficacy on brightness was carried out by statistical analysis of the readings taken by all volunteers before applying the product (T0), after 14 days (T14D), and at 28 days after applying the product (T28D). The individual results were expressed in absolute values for each time and in percentage of variation between the times T0-T14D and T0-T28D.

Means and standard deviation were calculated. The results are summarized in the following table (Table 3):

**Table 3: Average results of skin radiance after comparing measurements taken before product application (T0), after 14 days (T14D) and after 28 days (T28D) of product use after a daily application.**

| | AVERAGE LUMINOSITY RESULTS | | | | |
|---|---|---|---|---|---|
| | Absolute values | | | % values | |
| | T0 | T14D | T28D | T0-T14D | T0-T28D |
| Average of individual results | 60,42 | 60,88 | 63,05 | 1% | 4% |
| % volunteers with global improvement | - | - | - | 53% | 100% |

The product, as seen in Table 3, had an effect between T0-T14D on the 30 volunteers of 1% and, after 28 days, the brightness level was increased by 4% among all volunteers.

### Example 7: Efficacy on elasticity and firmness of the skin

The same single-centre open-label study with dermatological and instrumental evaluation as described for Examples 5 and 6 was used to measure efficacy of the composition herein disclosed on elasticity and firmness of the skin. The subjects remained resting for approximately 20 minutes in an air-conditioned room, after which the test began. Both the resting room and the testing room where the test was carried out have regulated and controlled conditions of temperature (20±2°C) and humidity (45±15%).

The resting period allowed each subject to relax, and the skin to reach a balance in relation to the humidity level. The study was carried out with a measuring device called Cutometer^{®} Dual MPA580 on facial skin, using vacuum pressure that deforms the skin mechanically after an adjustable suction time; the vacuum pressure was set to zero, making it possible to visualize the changes in skin elasticity and firmness.

This measuring equipment consists of a light source and a light receiver as well as two prisms facing each other, which project the light from the transmitter to the receiver. The intensity of the light varies due to the depth of penetration into the skin. The results assess the skin's resistance to negative pressure: firmness (that is why firmness is provided with negative figures) and its ability to return to its original position (elasticity). A negative firmness value with this test means that better firmness is provided, thus an improvement is achieved. Bear in mind that in the literature sometimes an improvement in firmness is also expressed using positive figures.

Measurements were carried out before application of the product T0, after 14 days (T14D) and 28 days (T28D) of regular use at the volunteers' home. The efficacy on the skin elasticity and firmness was evaluated quantitatively using the equipment Cutometer Dual MPA 580 to perform instrumental measurements on volunteers' skin before, after 14 days and after 28 days of use of the tested product. Measurements were taken on the area where the product was applied (face).

The results are summarized in the following table (Table 4):

**Table 4: Average results obtained for skin firmness and elasticity with measurements taken before (T0), after 14 days (T14D) and after 28 days (T28D) of use of the product.**

| | Parameter | T0 | T14D | T28D | T0-T14D | T0-T28D |
|---|---|---|---|---|---|---|
| Firmness | R0 | 0,318 | 0,251 | 0,205 | -21% | -36% |
| Gross elasticity | R2 | 40,242 | 42,603 | 47,903 | 6% | 19% |
| Neat elasticity | R5 | 41,907 | 44,292 | 51,623 | 6% | 23% |
| Elasticity over the entire surface | R7 | 32,998 | 37,924 | 48,303 | 15% | 46% |
| Average elasticity | - | 38,386 | 41,606 | 49,276 | 8% | 28% |
| % volunteers with global improvement | - | - | - | - | 40% | 100% |

Therefore, as shown in table 4, after 28 days of use, an average decrease in skin firmness and an average increase in the parameters assessed for elasticity are observed, both indicating an improvement in skin condition.

After 14 days, for volunteers with a positive reaction, the mean improvement in firmness was - 21%, the average elasticity was 8% and the elasticity over the entire surface improved 15%. After 28 days, for volunteers with a positive reaction, the average improvement in firmness is - 36%, the average elasticity was 28% and the elasticity over the entire surface improved 46 %.

Therefore, the results obtained from the studies with volunteers (Examples 5, 6 and 7) concluded that the composition as disclosed herein was well tolerated by the skin, had an antiwrinkle effect reducing volume, area and depth, improved skin luminosity and had a statistically significant positive effect on firmness and elasticity of the skin.

Furthermore, the compositions of the invention have shown better elasticity and firmness properties when compared to those of the prior-art. For instance, the antioxidant cream described in the anonymous publication entitled "3X Antioxidant Cream" cited moderate improvements in elasticity, of 12.0% (around the cheeks/mouth area), 17.2% (2mm in-depth inner elasticity) or 6.98% improvement in elasticity up to the 15th layer of the skin. All these improvements achieved were lower than those obtained for the composition of the invention, where the improvement in elasticity ranged from 19 to 46%, being the improvement of the elasticity over the entire surface of 46%.

When compared to the anonymous internet publication "Retinol Super Bounce Serum", the serum composition disclosed therein demonstrated a modest increase in skin firmness of approximately 12.2%. This value is notably lower than the improvement achieved by the composition of the present invention, which showed a 36% increase in skin firmness. Notably, despite the higher concentrations of retinol precursors and retinal in the prior art composition compared to the compositions of the invention, the invention still provided a significantly greater improvement.

Additionally, the study mentioned in the anonymous internet publication "skin Retinol Super Bounce Serum", disclosed that the participants reported a reduction of -18.0% in pore size and - 21.3% in pore number, where in the study carried out with the composition of the invention the participants reported better results, as the compositions of the invention provided a 70% spot reduction and 87% better appearance of the pores on the skin.

## Claims

1. Cosmetic composition for rejuvenating the skin comprising:
- from 0,01% to 0,5% by weight with respect to the total weight of the composition of a precursor of retinoic acid which is retinaldehyde,
- from 0,0001% to 0,01% by weight with respect to the total weight of the composition of an anti-aging or antioxidant peptide, and
- from 0,0000005% to 0,000005% by weight with respect to the total weight of the composition of a growth factor,
wherein the precursor of retinoic acid is encapsulated by an encapsulating agent comprising phospholipids.

2. The cosmetic composition according to claim 1, wherein the anti-aging or antioxidant peptide is selected from the group consisting of carnosine, copper tripeptide-1, pentapeptide-18, palmitoyl tetrapeptide-7, palmitoyl pentapeptide-4, nonapeptide-1, hexapeptide-2, acetyl hexapeptide-8, acetyl octapeptide-3, acetyl hexapeptide-1 o acetyl tetrapeptide-2, and combinations thereof.

3. The cosmetic composition according to claim 2, wherein the anti-aging or antioxidant peptide is acetyl tetrapeptide-2.

4. The cosmetic composition according to any one of the previous claims, wherein the anti-aging or antioxidant peptide is in an amount of from 0,0005% to 0,005% by weight with respect to the total weight of the composition.

5. The cosmetic composition according to any one of the previous claims, wherein the growth factor is selected from the group consisting of epidermal growth factor, insulin-like growth factor 1, transforming growth factor beta-2, Nicotiana Benthamiana Hexapeptide-40 sh-Polypeptide-76, sh-Polypeptide-1, sh-Polypeptide-9, sh-Polypeptide-11, Oligopeptide-4, Oligopeptide-2, Oligopeptide-1, and combinations thereof.

6. The cosmetic composition according to claim 5, wherein the growth factor is Oligopeptide-1, epidermal growth factor, and/or combinations thereof.

7. The cosmetic composition according to any one of the previous claims, wherein the growth factor is in an amount of from 0,000001% to 0,000003% by weight with respect to the total weight of the composition.

8. The cosmetic composition according to any one of the previous claims, wherein the phospholipids are derived from lecithin.

9. The cosmetic composition according to any one of the previous claims, wherein the phospholipids are in an amount of from 0,1% to 2% by weight with respect to the total weight of the composition.

10. The cosmetic composition according to claim 1, wherein the anti-aging or antioxidant peptide is not acetyl tetrapeptide-11.

11. The cosmetic composition according to any one of the previous claims, wherein the encapsulating agent is a liposome, preferably wherein the liposome comprises one lipidic bilayer.

12. The cosmetic composition according to any one of the previous claims, wherein the retinol precursor consists of retinal.

13. The cosmetic composition according to any one of the previous claims, further comprising an emollient or wherein the cosmetic composition further comprises an emollient selected from the group consisting of squalene, sweet almond oil and combinations thereof.

14. Method for rejuvenating the skin, **characterized in that** it comprises the topical application of the cosmetic composition according to any of the claims 1 to 13.

15. Use of the cosmetic composition according to any of the claims 1 to 13 for rejuvenating the skin, by means of its topical application.
